# EUROPEAN PATENT SPECIFICATION

(11) **EP 1 097 136 B1**
(45) Date of publication and mention of the grant of the patent: **22.10.2003**
(21) Application number: 99938234.4
(22) Date of filing: 13.07.1999
(51) Int. Cl.: C07D 209/42

(54) **PROCESS FOR THE PREPARATION OF AN INDOLE DERIVATIVE**
VERFAHREN ZUR HERSTELLUNG EINES INDOLDERIVATES
PROCEDE DE PREPARATION D'UN DERIVE D'INDOLE

(30) Priority: 16.07.1998 GB 9815481
(43) Date of publication of application: 09.05.2001
(73) Proprietor: SmithKline Beecham plc, Brentford, Middlesex TW8 9GS (GB)
(72) Inventor: FEDOULOFF, Michael, SmithKline Beecham Pharma., Harlow, Essex CM19 5AW (GB); STRACHAN, John Bryce, SmithKline Beecham Pharma., Harlow, Essex CM19 5AW (GB)
(74) Representative: Waters, David Martin
(86) International application number: EP9904944
(87) International publication number: WO00003984

(56) References cited:
- WO-A-98/07728
- E.P. SERGEANT, BOYD DEMPSEY: "IUPAC CHEMICAL DATA SERIES- No.23 IONISATION CONSTANTS OF ORGANIC ACIDS IN AQUEOUS SOLUTION", 1979, PERGAMON PRESS, GREAT BRITAIN

## Description

This invention relates to a new synthetic process to a compound having pharmacological activity.

WO 93/18036 (SmithKline Beecham plc) describes certain indole compounds having 5-HT₄ receptor antagonist activity including the compound of formula (I) 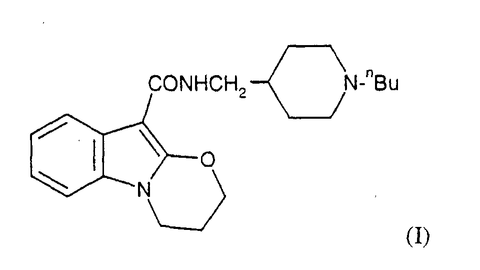 and its pharmaceutically acceptable salts. This compound is N-[(1-ⁿbutyl-4-piperidyl)methyl]-3,4-dihydro-2H-[1,3]oxazino[3,2-a]indole-10-carboxamide, referred to herein by its code number SB-207266, (the hydrochloride salt is SB-207266-A), which is being developed by SmithKline Beecham plc as the active ingredient in a medicament for treatment of irritable bowel syndrome.

Example 3 of WO 93/18036 describes a method of preparation of SB-207266-A from N-[(1-ⁿbutyl-4-piperidyl)methyl]indole-3-carboxamide (i.e. the compound corresponding to SB-207266, without the oxazino moiety), by reacting with N-chlorosuccinimide and 3-bromo-1-propanol, followed by treatment with sodium carbonate. N-[(1-ⁿbutyl-4-piperidyl)methyl]indole-3-carboxamide is prepared by coupling N-(1-ⁿbutyl-4-piperidyl)methylamine with a indole-3-carboxylic acid.

WO 98/07728 (SmithKline Beecham plc) describes a process for preparing SB-207266-A which involves the use of the N-(1-ⁿbutyl-4-piperidyl)methylamine intermediate at a later stage in the process thus resulting in an increased yield of SB-207266-A relative to the amount of this intermediate, which is relatively expensive to produce. In particular, the alternative process comprises the reaction of of N-(1-ⁿbutyl-4-piperidyl)methylamine with a compound of formula (A): 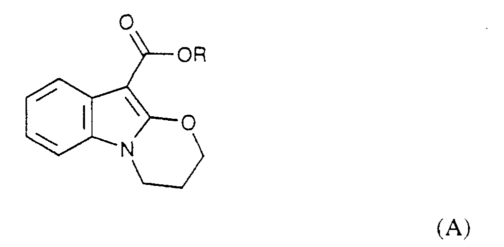 wherein R is alkyl, such as methyl or ethyl.

The compound of formula (A) wherein R is methyl is methyl 3,4-dihydro-2*H*-[1,3]-oxazino[3,2-*a*]indole-10-carboxylate.

WO98/07728 also describes the preparation of the oxazinoindole compound of the formula (A) from the corresponding indole by reaction with N-chlorosuccinimide and a 3-halo-propanol, such as 3-chloropropanol or 3-bromopropanol followed by cyclisation of the intermediate (B) by treatment with base in a suitable solvent.

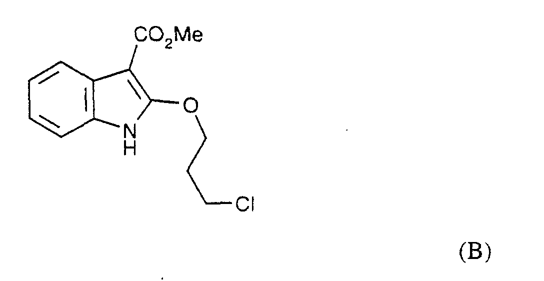

The Description in the latter specification describes in more detail the the preparation of compound (B) from the corresponding methyl indole-3-carboxylate by reaction of the latter with N-chlorosuccinimide in the presence of 1,4-diazabicyclo[2.2.2]octane (DABCO) to form an intermediate of formula (C): 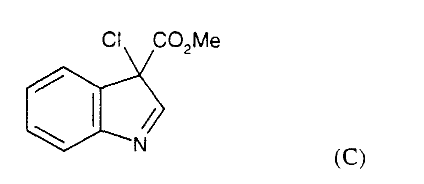 and subsequent reaction of (C) with 3-chloropropanol in the presence of methanesulphonic acid.

We have now found that the use of an acid having a pKa of from 0 to 2, especially trichloroacetic acid, in place of methanesulphonic acid results in significant advantages for the commercial operation of the process.

According to a feature of the present invention we provide a process for the preparation of the compound of formula (B) above, namely methyl 2-(3-chloropropyl-1-oxy)-indole-3-carboxylate, which comprises reacting a compound of formula (C) with 3-chloropropan-1-ol in the presence of an acid having a pKa of from 0 to 2, especially trichloroacetic acid.

Other acids which may be used in accordance with the invention in addition to trichloroacetic acid include dichloroacetic acid and trifluoroacetic acid.

The use of the above-defined acid such as trichloroacetic acid in place of methanesulphonic acid has been found to increase significantly the overall yield of the process. The former acid also has the advantage over the latter that its use results in the formation of lower levels of the corresponding 2-methoxy compound, as an impurity.

The reaction is conveniently effected in an organic solvent such as dichloromethane or chloroform, at a temperature in the range -20°C to +10°C , for example using a catalytic amount of the acid. The resulting product of formula (B) can be used for the next stage in the synthesis of SB-207266 e.g as described in WO 98/07728.

The following Example illustrates the invention.

### Example

### Methyl 2-(3-chloropropyl-1-oxy)-indole-3-carboxylate ( formula (B))

A mixture of methyl indole-3-carboxylate and dichloromethane is cooled to 0° C. 1,4-dimethylpiperazine (0.55eq.) and N-chlorosuccinimide (1.1 eq) are added and the mixture left to stir for two hours to give a slurry containing the compound of formula (C) above. The resulting slurry is added to a solution of 3-chloropropan-1-ol (1.1 eq) and trichloroacetic acid (0.12 eq) in dichloromethane, maintaining the temperature below 0° C. The reaction mixture is left to stir for half an hour, then washed with 10% aqueous sodium carbonate, 0.5 M hydrochloric acid and water. The organic solution is dried over sodium sulphate, filtered and the solvent evaporated. Toluene is added and the mixture stirred at 0-5° C for one hour. The product is then filtered, washed with toluene and dried to give the title product in 83 % yield.

## Claims

1. A process for the preparation of methyl 2-(3-chloropropyl-1-oxy)-indole-3-carboxylate, i.e. the compound of formula (B): 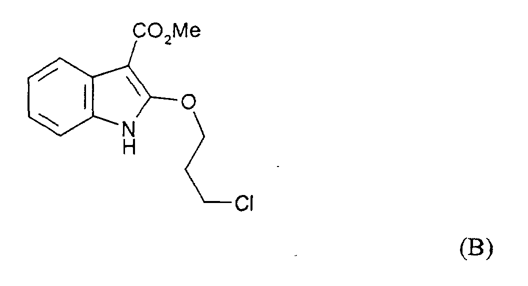 which process comprises reacting a compound of formula (C) 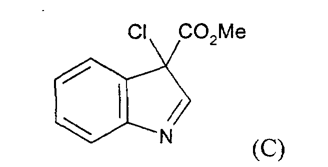 with 3-chloropropan-1-ol in the presence of an acid having a pKa of from 0 to 2.

2. A process as claimed in claim 1 in which the acid is trichloroacetic acid, dichloroacetic acid and/or trifluoroacetic acid.

3. A process as claimed in claim 1 in which the acid is trichloroacetic acid.

4. A process as claimed in claim 1, 2 or 3 in which the reaction is effected in an organic solvent at a temperature in the range -20°C to +10°C.

5. A process as claimed in claim 4 in which the organic solvent is dichloromethane or chloroform.

6. A process as claimed in claim 4 or 5 wherein the reaction is effected using a catalytic amount of the acid.

7. A process for preparing methyl 3,4-dihydro-2*H*-[1,3]-oxazino[3,2-*a*]indole-10-carboxylate, comprising performing a process as defined in any one of claims 1 to 6 followed by cyclising the intermediate (B) (the methyl 2-(3-chloropropyl-1-oxy)-indole-3-carboxylate) by treatment with base in a suitable solvent.

8. A process for preparing N-[(1-ⁿbutyl-4-piperidyl)methyl]-3,4-dihydro-2H-[1,3]oxazino[3,2-a]indole-10-carboxamide, i.e. the compound of Formula (I): 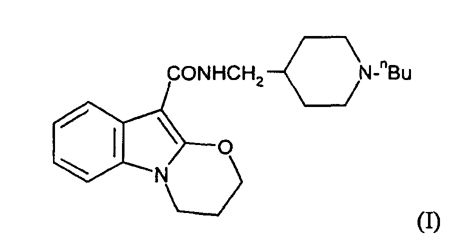 , or a pharmaceutically acceptable salt thereof, comprising performing a process as defined in any one of claims 1 to 6 and using the resulting methyl 2-(3-chloropropyl-1-oxy)-indole-3-carboxylate of formula (B) in the next stage in the synthesis of the N-[(1-ⁿbutyl-4-piperidyl)methyl]-3,4-dihydro-2H-[1,3]oxazino[3,2-a]indole-10-carboxamide or said salt thereof.

9. A process as claimed in claim 8 comprising cyclising the intermediate (B) (the methyl 2-(3-chloropropyl-1-oxy)-indole-3-carboxylate) by treatment with base in a suitable solvent to prepare methyl 3,4-dihydro-2*H*-[1,3]-oxazino[3,2-*a*]indole-10-carboxylate.

10. A process as claimed in claim 8, comprising
(i) preparing methyl 3,4-dihydro-2*H*-[1,3]-oxazino[3,2-*a*]indole-10-carboxylate by cyclisation of the intermediate (B) (the methyl 2-(3-chloropropyl-1-oxy)-indole-3-carboxylate) by treatment with base in a suitable solvent, and
(ii) reacting N-(1-ⁿbutyl-4-piperidyl)methylamine with the methyl 3,4-dihydro-2*H*-[1,3]-oxazino[3,2-*a*]indole-10-carboxylate.

11. A process as claimed in claim 8, 9 or 10, in which the hydrochloride salt of N-[(1-ⁿbutyl-4-piperidyl)methyl]-3,4-dihydro-2H-[1,3]oxazino[3,2-a]indole-10-carboxamide (SB-207266-A) is prepared.

## Patentansprüche

1. Verfahren zur Herstellung von Methyl-2(3-chlorpropyl-1-oxy)-indol-3-carboxylat, nämlich der Verbindung von Formel (B): 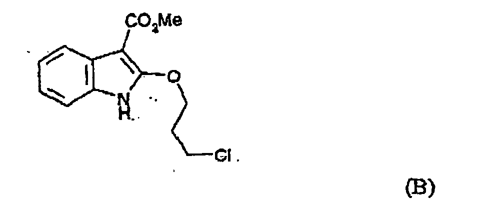 wobei das Verfahren umfaßt, eine Umsetzung der Verbindung der Formel (C): 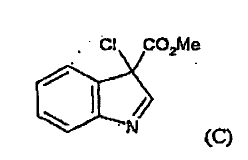 mit 3-Chlorpropan-1-ol in Gegenwart einer Säure mit einem pKa-Wert von 0 bis 2.

2. Verfahren nach Anspruch 1, wobei die Säure Trichloressigsäure, Dichloressigsäure und/oder Trifluoressigsäure ist.

3. Verfahren nach Anspruch 1, bei dem die Säure Trichloressigsäure ist.

4. Verfahren nach Anspruch 1, 2 oder 3, wobei die Umsetzung in einem organischen Lösungsmittel bei einer Temperatur im Bereich von -20 bis +10°C durchgeführt wird.

5. Verfahren nach Anspruch 4, bei dem das organische Lösungsmittel Dichlormethan oder Chloroform ist.

6. Verfahren nach Anspruch 4 oder 5, wobei die Umsetzung unter Verwendung einer katalytischen Menge der Säure durchgeführt wird.

7. Verfahren zur Herstellung von Methyl-3,4-dihydro-2*H*-[1,3]-oxazino[3,2-a]indol-10-carboxylat, umfassend die Durchführung eines Verfahrens gemäß einem der Ansprüche 1 bis 6, gefolgt von einer Cyklisierung des Zwischenprodukts (B) (dem Methyl-2-(3-chlorpropyl-1-oxy)-indol-3-carboxylat) durch Behandlung mit einer Base in einem geeigneten Lösungsmittel.

8. Verfahren zur Herstellung von N-[(1-n-butyl-4-piperidyl)methyl]-3,4-dihydro-2H-[1,3]oxazino[3,2-a]indol-10-carboxamid, nämlich der Verbindung von Formel (I): 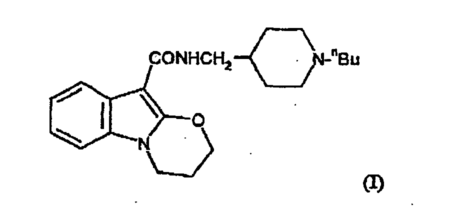 , oder eines pharmazeutisch verträglichen Salzes davon, umfassend die Durchführung eines Verfahrens gemäß einem der Ansprüche 1 bis 6, und wobei man das entstandene Methyl-2-(3-chlorpropyl-1-oxy)-indol-3-carboxylat der Formel (B) in der nächsten Stufe bei der Synthese von N-[(1-n-butyl-4-piperidyl)methyl]-3,4-dihydro-2H-[1,3]oxazino[3,2-a]indol-10-carboxamid oder von dessen Salz verwendet.

9. Verfahren nach Anspruch 8, umfassend das Cyklisieren des Zwischenprodukts (B) (dem Methyl-2-(3-chlorpropyl-1-oxy)-indol-3-carboxylat) durch Umsetzung mit einer Base in einem geeigneten Lösungsmittel, um Methyl-3,4-dihydro-2H-[1,3]-oxazino[3,2-a]indol-10-carboxylat zu ergeben.

10. Verfahren nach Anspruch 8, umfassend
(i) Herstellung von Methyl-3,4-dihydro-2H-[1,3]-oxazino[3,2-a]indol-10-carboxylat durch Cyklisieren des Zwischenprodukts (B) (dem Methyl-2-(3-chlorpropyl-1-oxy)-indol-3-carboxylat) durch Umsetzung mit einer Base in einem geeigneten Lösungsmittel, und
(ii) Umsetzung von N-(1-n-butyl-4-piperidyl)methylamin mit dem Methyl-3,4-dihydro-*2H*-[1,3]-oxazino[3,2-a]indol-10-carboxylat.

11. Verfahren nach Anspruch 8, 9 oder 10, zur Herstellung des Hydrochlorid-Salzes von N-[(1-n-butyl-4-piperidyl)-methyl]-3,4-dihydro-2H-[1,3]oxazino[3,2-a]indol-10-carboxamid (SB-207266-A).

## Revendications

1. Procédé pour la préparation du 2-(3-chloropropyl-1-oxy)-indole-3-carboxylate de méthyle, c'est-à-dire du composé de formule (B) : 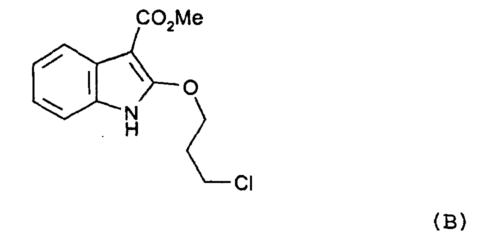 procédé qui comprend la réaction d'un composé de formule (C) 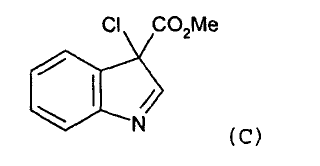 avec du 3-chloropropane-1-ol en présence d'un acide ayant un pKa de 0 à 2.

2. Procédé suivant la revendication 1, dans lequel l'acide est l'acide trichloracétique, l'acide dichloracétique et/ou l'acide trifluoracétique.

3. Procédé suivant la revendication 1, dans lequel l'acide est l'acide trichloracétique.

4. Procédé suivant la revendication 1, 2 ou 3, dans lequel la réaction est conduite dans un solvant organique à une température comprise dans l'intervalle de -20°C à +10°C.

5. Procédé suivant la revendication 4, dans lequel le solvant organique est le dichlorométhane ou le chloroforme.

6. Procédé suivant la revendication 4 ou 5, dans lequel la réaction est conduite en utilisant une quantité catalytique de l'acide.

7. Procédé pour la préparation du 3,4-dihydro-2*H*-[1,3]-oxazino[3,2-*a*]indole-10-carboxylate de méthyle, comprenant la mise en oeuvre d'un procédé répondant à la définition suivant l'une quelconque des revendications 1 à 6 et ensuite la cyclisation de l'intermédiaire (B) (le 2-(3-chloropropyl-1-oxy)-indole-3-carboxylate de méthyle) par traitement avec une base dans un solvant convenable.

8. Procédé pour la préparation du N-[(1-ⁿbutyl-4-pipéridyl)méthyle]-3,4-dihydro-2H-[1,3]oxazino[3,2-a]indole-10-carboxamide, c'est-à-dire du composé de formule (I) : 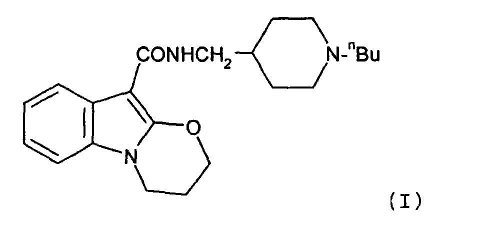 , ou d'un de ses sels pharmaceutiquement acceptables, comprenant la mise en oeuvre d'un procédé répondant à la définition suivant l'une quelconque des revendications 1 à 6 et l'utilisation du 2-(3-chloropropyl-1-oxy)-indole-3-carboxylate de méthyle résultant de formule (B) dans l'étape suivante dans la synthèse du N-[(1-ⁿbutyl-4-pipéridyl)méthyle]-3,4-dihydro-2H-[1,3]oxazino[3,2-a]indole-10-carboxamide ou de son sel.

9. Procédé suivant la revendication 8, comprenant la cyclisation d'un intermédiaire (B) (le 2-(3-chloropropyl-1-oxy)-indole-3-carboxylate de méthyle) par traitement avec une base dans un solvant convenable pour préparer le 3,4-dihydro-2*H*-[1,3]-oxazino[3,2-*a*]indole-10-carboxylate de méthyle.

10. Procédé suivant la revendication 8, comprenant
(i) la préparation de 3,4-dihydro-2*H*-[1,3]-oxazino[3,2-*a*]indole-10-carboxylate de méthyle par cyclisation d'un intermédiaire (B) (le 2-(3-chloropropyl-1-oxy)-indole-3-carboxylate de méthyle) par traitement avec une base dans un solvant convenable, et
(ii) la réaction de la N-(1-ⁿbutyl-4-pipéridyl)méthylamine avec le 3,4-dihydro-2*H*-[1,3]-oxazino[3,2-*a*]indole-10-carboxylate de méthyle.

11. Procédé suivant la revendication 8, 9 ou 10, dans lequel le chlorhydrate du N-[(1-ⁿbutyl-4-pipéridyl)méthyl]-3,4-dihydro-2H-[1,3]oxazino[3,2-a]indole-10-carboxamide (SB-207266-A) est préparé.
